# EUROPÄISCHE PATENTSCHRIFT

(11) **EP 1 066 240 B1**
(45) Veröffentlichungstag und Bekanntmachung des Hinweises auf die Patenterteilung: **06.03.2002**
(21) Anmeldenummer: 99913295.4
(22) Anmeldetag: 24.03.1999
(51) Int. Cl.: C07C 51/21, C07C 51/44, C07C 57/05, C07C 67/08, C07C 69/54

(54) **VERFAHREN ZUR HERSTELLUNG VON ACRYLSÄURE UND ACRYLSÄUREESTERN**
METHOD FOR PRODUCING ACRYLIC ACID AND ACRYLIC ACID ESTERS
PROCEDE DE PREPARATION D'ACIDE ACRYLIQUE ET D'ESTERS D'ACIDE ACRYLIQUE

(30) Priorität: 31.03.1998 DE 19814375
(43) Veröffentlichungstag der Anmeldung: 10.01.2001
(73) Patentinhaber: BASF AKTIENGESELLSCHAFT, 67056 Ludwigshafen (DE)
(72) Erfinder: NESTLER, Gerhard, D-67061 Ludwigshafen (DE); SCHRÖDER, Jürgen, D-67071 Ludwigshafen (DE); BOLZ, Gerhard, D-67227 Frankenthal (DE)
(74) Vertreter: Isenbruck, Günter, Dr.
(86) Internationale Anmeldenummer: EP9901998
(87) Internationale Veröffentlichungsnummer: WO9950220

(56) Entgegenhaltungen:
- EP-A- 0 398 226
- DE-A- 1 910 795
- DE-A- 2 136 396
- DE-A- 19 740 253
- US-A- 3 555 082

## Beschreibung

Die vorliegende Erfindung betrifft ein Verfahren zur Herstellung von Acrylsäure, in dem ein bei der Gasphasenoxidation zur Herstellung von Acrylsäure anfallendes gasförmiges Reaktionsgemisch, das Acrylsäure enthält, mittels eines inerten hochsiedenden Lösungsmittels abgekühlt wird, und ein Acrylsäure enthaltendes gasförmiges Gemisch erhalten wird. Darüber hinaus betrifft sie ganz allgemein die Verwendung eines inerten hochsiedenden Lösungsmittels zum Abkühlen eines bei der Gasphasenoxidation zur Herstellung von Acrylsäure anfallenden gasförmigen Reaktionsgemisches, sowie ein Verfahren zur Herstellung von Acrylsäureestern.

Acrylsäure bildet aufgrund ihrer sehr reaktionsfähigen Doppelbindung sowie der Säurefünktion ein wertvolles Monomeres zur Herstellung von Polymerisaten, z.B. für als Klebstoffe geeignete wäßrige Polymerisatdispersionen.

Unter anderem ist Acrylsäure zugänglich durch Gasphasenoxidation von Propylen und/oder Acrolein mit Sauerstoff oder Sauerstoff enthaltenden Gasen in Gegenwart von Katalysatoren bei erhöhter Temperatur, infolge der hohen Reaktionswärme vorzugsweise unter Verdünnen der Reaktionspartner mit Inertgasen und/oder Wasserdampf.

Als Katalysatoren werden dabei in der Regel oxidische Mehrkomponentensysteme, z.B. auf Basis von Molybdän-, Chrom-, Vanadium- oder Telluroxiden, eingesetzt.

Bei diesem Verfahren wird jedoch nicht reine Acrylsäure, sondern ein Gasgemisch, das neben Acrylsäure als Nebenkomponenten im wesentlichen nicht umgesetztes Acrolein und/oder Propylen, Wasserdampf, Kohlenoxide, Stickstoff, Sauerstoff, Essigsäure, Formaldehyd, Benzaldehyd, Furfurale und Maleinsäureanhydrid enthält, erhalten. Aus diesem Gasgemisch muß die Acrylsäure anschließend abgetrennt werden.

Die Isolierung der Acrylsäure aus dem gasförmigen Reaktionsgemisch erfolgt in der Regel durch Gegenstromabsorption z.B. mit einem hochsiedenden Lösungsmittel bzw. Lösungsmittelgemisch und mehreren anschließenden destillativen Aufarbeitungsschritten. wie dies z.B. in der DE-A 21 36 396 und der DE-A 43 08 087 beschrieben wird. Gemäß der EP-B 0 009 545, US 5 154 800, DE-A 34 29 391 sowie der DE-A 21 21 123 wird zunächst mit Wasser/wäßriger Acrylsäure im Gegenstrom absorbiert und anschließend extraktiv oder azeotrop destilliert.

Nachteilig bei diesen Verfahren ist es, daß das zur Absorption bzw. Extraktion verwendete organische Lösungsmittel/Lösungsmittelgemisch in einem eigenen Destillationsschritt wieder abgetrennt und gegebenenfalls vor der Wiederverwendung gereinigt werden muß.

Ein weiterer Nachteil dieser Verfahren besteht darin, daß die bei der Acrylsäure-Herstellung anfallende Essigsäure (Gehalt: 0,5 bis 10 Gew.-% bezogen auf die Menge der Acrylsäure) in einer aufwendigen Destillationsstufe abgetrennt werden muß. Aufgrund der geringen Siedepunktsunterschiede und der hohen Polymerisationsneigung der Acrylsäure erfordert dies i.d.R. mehrere Destillationsschritte, wie sich u.a. der US 3 844 903 entnehmen läßt, und verursacht beträchtliche Verluste an Acrylsäure (vgl. EP-A 398 226).

Im Hinblick auf die bekannte Tatsache, daß Acrylverbindungen eine große Neigung zur Polymerisation besitzen, sind ganz allgemein Verfahren mit mehrstufiger destillativer Aufarbeitung nachteilig, da sie die Polymerisationstendenz der Acrylsäure noch verstärken.

Auch die Herstellung von Acrylsäureestern durch säurekatalysierte Veresterung von Acrylsäure mit einem oder mehreren Alkanolen ist aus dem Stand der Technik bekannt. Bzgl. derartiger Veresterungsreaktionen ist ganz allgemein bekannt, daß es sich um Gleichgewichtsreaktionen handelt und von daher die Anwesenheit von Wasser im Reaktionsgleichgewicht wirtschaftliche Umsätze verhindert. Demgemäß wird i.d.R. weitgehend wasserfreie Acrylsäure eingesetzt und das bei der Veresterung entstehende Reaktionswasser, ggf. mit Hilfe eines Schleppmittels destillativ entfernt.

Wie bereits eingangs erwähnt, entstehen bei der Herstellung von Acrylsäure durch Oxidation ausgehend von den entsprechenden C₃-Vorläufern noch beträchtliche Mengen an Essigsäure (0,5 bis 10 Gew.-%). Aufgrund der zum Teil geringen Siedepunktsunterschiede und der hohen Polymerisationsneigung der Acrylsäure bei thermischer Belastung, ist eine destillative Abtrennung der o.g. Nebenprodukte schwierig und aufwendig (US 3 844 903, DE-A 2 164 767).

Da bei der Veresterung von essigsäurehaltiger Acrylsäure mit Alkanolen auch die Essigsäure verestert wird, bedingt die Bildung des Essigesters einen zusätzlichen Trennaufwand und einen Verlust an Alkanol. Dabei ist ferner zu beachten, daß die destillative Abtrennung des Essigsäurealkylesters aus dem Veresterungsgemisch, vor allem die Trennung von nicht umgesetztem Alkanol, wegen der Ausbildung von binären Azeotropen, behindert wird.

Im Falle von z.B. Butanol siedet das Butanol-Butylacetat-Azeotrop bei 115,8 °C (57 % Butanol), wobei Butanol bei 117,4 °C und Butylacetat bei 125,6 °C sieden.

Da die Essigsäureester relativ leicht flüchtig und nicht polymerisierbar sind, werden bei der Herstellung von Polymerisaten i.d.R. hochreine Acrylsäureester, d.h.

Acrylsäureester, die möglichst, d.h. im wesentlichen, frei von Essigsäureestern sind, benötigt. Der beispielsweise in einer Anstrichdispersion oder in einem Klebstoff verbleibende Essigsäureester würde nämlich u.a. eine starke Geruchsbelästigung hervorrufen. Eine aufwendige Entfernung (Desodorierung) des Essigsäureesters wäre notwendig.

Wie sich aus obigem ergibt, besteht bei der Acrylsäureester-Herstellung prinzipiell das Problem eines zu hohen Verbrauchs an Alkanolen, was sowohl wirtschaftlich als auch ökologisch nachteilig ist.

Es wurden daher in der Vergangenheit bereits verschiedene Versuche unternommen, die Probleme, die die bei der Synthese von Acrylsäure durch Gasphasenoxidation anfallenden Nebenprodukte, wie z.B. Essigsäure und Wasser bei der Veresterung mit Alkanolen verursachen, zu lösen.

So beschreibt die DE-A 20 35 228 die Veresterung von wäßriger Acrylsäure (Gehalt an Wasser: mindestens 30 %) in Gegenwart von sauren Katalysatoren, wie z.B. Schwefel-, Sulfon-, Phosphorsäure und einem organischem Lösungsmittelgemisch.

Dieses Verfahren ist insbesondere dahingehend nachteilig, daß bedingt durch die große Menge an Wasser, die zu einer Verringerung der Katalysatorkonzentration führt, eine große Menge an Katalysator verwendet werden muß. Um brauchbare Umsätze bzw. Veresterungsgeschwindigkeiten zu erreichen, müssen darüber hinaus gemäß dieser Druckschrift spezielle Lösungsmittelgemische, bestehend aus einem aromatischen und einem aliphatischen Kohlenwasserstoff verwendet werden. Eine weitere Bedingung zur erfolgreichen Durchführung dieses Verfahrens liegt darin, daß das Lösungsmittelgemisch außerdem deutlich höher sieden muß als der Acrylsäureester.

Die EP-A 0 398 226 schlägt vor, eine partielle Kondensation der Reaktionsgase der Propylenoxidation durchzuführen und die dabei anfallende Acrylsäure (enriched acylic acid) direkt zu verestern und die im Reaktionsgas verbleibende Acrylsäure auf herkömmliche Weise mit Wasser auszuwaschen und destillativ abzutrennen. Dieses Verfahren der zweistufigen Acrylsäurekondensation ist sehr aufwendig und liefert keine essigsäurefreie bzw. -arme Acrylsäure. Wie die dort wiedergegebenen Beispiele zeigen, enthalten die erhaltenen Veresterungsgemische noch 1,8 bis 2,5 Gew.- % Essigsäureester.

Gemäß der DE-A 1 668 362 wird das acrylsäurehaltige Reaktionsgas der Propylenoxidation mit den bei der Veresterung anfallenden Hochsiedergemischen, die im wesentlichen aus Maleinsäureester, Polyacrylsäure und Polyacrylsäureestern bestehen, behandelt und die dabei anfallende Acrylsäurelösung destillativ von den Leichtsiedern befreit. Die so erhaltene acrylsäurehaltige Sumpflösung wird mit Alkanolen in Gegenwart von sauren Kationenaustauschern verestert.

Nachteilig bei diesem Verfahren ist, daß die Acrylsäureaufarbeitung mit der Esterherstellung gekoppelt ist, wobei man die Möglichkeit zur Herstellung verschiedener Ester verliert. Darüber hinaus werden bedingt durch die Bildung von hochsiedenden Estern der Polyacrylsäure, deren Überschuß entsorgt werden muß, beträchtliche Alkanolverluste verursacht.

Die JA 7 014 529-R beschreibt die Herstellung von Butylacrylat aus wäßriger Acrylsäure. Gemäß des darin beschriebenen Verfahrens wird Acrylsäure mit einem Butylacrylat-Butanol-Gemisch aus der wäßrigen Lösung extrahiert und anschließend das Extrakt, das ca. 18 Gew.-% Acrylsäure, ca. 1 Gew.-% Essigsäure und ca. 11 Gew.-% Wasser enthält, mit einem Alkanol verestert.

Nachteilig ist bei diesem Verfahren vor allem, daß große Wasser- und Essigsäuremengen in die Veresterung eingeschleust werden, was die Veresterung negativ beeinflußt und die Aufarbeitung des Veresterungsgemisches stört und Alkanolverluste verursacht.

Die FR-B 1 452 566 betrifft die Extraktion von Acrylsäure aus einer wäßrigen Lösung mit Acetophenon oder Tributylphosphat und die Veresterung dieser Acrylsäure mit überschüssigem Alkohol in Anwesenheit eines Extraktionsmittels. Die Ausbeute an Acrylsäureester gemäß dieses Verfahrens beträgt jedoch weniger als 80 %, bezogen auf die in der Ausgangslösung vorhandene Acrylsäure.

Aufgrund der negativen Auswirkung, die die Anwesenheit nennenswerter Mengen an Wasser und Essigsäure auf die Herstellung von Acrylsäureestern besitzt, wird i.d.R. wasserfreie und gereinigte Acrylsäure, die lediglich Spuren an Essigsäure enthält, zur Herstellung von Acrylsäureestern verwendet.

Eine Aufgabe der vorliegenden Erfindung besteht darin, ein einfaches Verfahren zur Gewinnung von wasserarmer Roh-Acrylsäure zur Verfügung zu stellen, das energetisch günstig durchführbar ist.

Eine weitere Aufgabe der vorliegenden Erfindung besteht darin, ein technisch einfaches und wirtschaftliches Verfahren zur Herstellung von Acrylsäureestern zur Verfügung zu stellen, bei dem trotz Anwesenheit von Essigsäure in der eingesetzten Acrylsäure die Alkanolverluste gering sind.

Demgemäß betrifft die vorliegende Erfindung ein Verfahren zur Herstellung von Acrylsäure, das folgende Stufe A umfaßt:
A: Abkühlen eines bei der Gasphasenoxidation zur Herstellung von Acrylsäure anfallenden gasförmigen Reaktionsgemisches, das Acrylsäure enthält, mittels eines inerten hochsiedenden Lösungsmittels, das hierbei nicht verdampft, wobei ein Acrylsäure enthaltendes, gasförmiges Gemisch erhalten wird.

Der Begriff "gasförmiges Reaktionsgemisch, das Acrylsäure enthält" umfaßt erfindungsgemäß alle Reaktionsgemische, die bei der Gasphasenoxidation zur Herstellung von Acrylsäure anfallen.

Hier erfolgt die Herstellung von Acrylsäure auf bekannte Weise an oxidischen Mehrkomponentenkatalysatoren bei ungefähr 200 bis 400 °C. Obwohl prinzipiell alle bekannten Reaktortypen verwendet werden, setzt man bevorzugt Rohrbündelwärmeaustauscher ein, die mit dem Oxidationskatalysator gefüllt sind. Der Grund hierfür liegt darin, daß der überwiegende Teil der bei der Oxidation freiwerdenden Wärme durch Konvektion und Strahlung an die gekühlten Rohrwände abgeführt werden kann.

Geht man von Propylen/Acrolein als Edukte zur Herstellung von Acrylsäure aus, handelt es sich um ein gasförmiges Reaktionsgemisch, das mit einer Temperatur von ungefähr 200 bis 300 °C aus der Gasphasenoxidation anfällt und ungefähr 1 bis ungefähr 30 Gew.-% Acrylsäure enthält und als Nebenprodukte nicht umgesetztes Propylen (ungefähr 0,05 bis ungefähr 1 Gew.-%), Acrolein (ungefähr 0,001 bis ungefähr 2 Gew.-%), Propan (ungefähr 0,01 bis ungefähr 2 Gew.-%), Wasserdampf (ungefähr 1 bis ungefähr 30 Gew.-%). Kohlenoxide (ungefähr 0,05 bis ungefähr 15 Gew.-%), Stickstoff (0 bis ungefähr 90 Gew.-%), Sauerstoff (ungefähr 0,05 bis ungefähr 10 Gew.-%), Essigsäure (ungefähr 0,05 bis ungefähr 2 Gew.-%), Propionsäure (ungefähr 0,01 bis ungefähr 2 Gew.-%), Aldehyde (ungefähr 0,05 bis ungefähr 3 Gew.-%) und Maleinsäureanhydrid (ungefähr 0,01 bis ungefähr 0,5 Gew.-%) umfaßt.

Dieses gasförmige Reaktiönsgemisch wird mittels eines inerten hochsiedenden Lösungsmittels, in der Regel auf eine Temperatur von ungefähr 100 bis ungefähr 190 °C, vorzugsweise ungefähr 120 bis ungefähr 180 °C und insbesondere ungefähr 130 °C bis ungefähr 160 °C abgekühlt, wobei ein wiederum gasförmiges, Acrylsäure enthaltendes Gemisch erhalten wird.

Der Begriff "inertes hochsiedendes Lösungsmittel" umfaßt Flüssigkeiten, deren Siedepunkt oberhalb des Siedepunkts der Acrylsäure, vorzugsweise über 160 °C bei 1 Atm. Druck liegt. Als Beispiele für derartige Lösungsmittel können z.B. Diphenyl, Diphenylether, Dimethylphthalat, Ethylhexansäure, N-Methylpyrrolidon, Fraktionen aus der Paraffindestillation oder Gemische aus zwei oder mehr davon, genannt werden.

Vorzugsweise wird das zur Abkühlung verwendete inerte hochsiedende Lösungsmittel im Kreis und insbesondere zwecks Wärmeabfluß über einen herkömmlichen Wärmeaustauscher gefahren.

Vorzugsweise wird dieses Lösungsmittel mit Stabilisatoren, wie z.B. Phenothiazin, Hydrochinon, einer phenolischen Verbindung oder einem Gemisch aus zwei oder mehr davon versetzt, wobei die Konzentration des Stabilisators bei 0,01 bis 1 Gew.-% liegt. Vorzugsweise werden Phenothiazin, Hydrochinon oder eine Gemisch aus Phenothiazin und einer N-O-Verbindung, wie z.B. p-Nitrosophenol, p-Nitrosodiethylanilin oder einem Tetramethyl-piperidin-1-oxyl als Stabilisatoren eingesetzt.

Als Abkühlvorrichtung können alle aus dem Stand der Technik für diesen Zweck bekannten Vorrichtungen eingesetzt werden, wobei vorzugsweise Venturi-Wäscher oder Sprühkühler (Quench) und insbesondere Sprühkühler verwendet werden.

Das so erhaltene Acrylsäure enthaltende gasförmige Gemisch wird vorzugsweise in einer Stufe B aufgetrennt, wobei eine Leichtsiederfraktion, eine Roh-Acrylsäure und ein Sumpfprodukt erhalten wird. Insbesondere wird die Auftrennung gemäß Stufe B in einer Destillationskolonne durchgeführt. Dabei wird das abgekühlte, Acrylsäure enthaltende gasförmige Gemisch in den unteren Teil der Destillationskolonne geleitet und die Destillation so durchgeführt, daß die gasförmigen Bestandteile und die Leichtsieder, d.h. hauptsächlich Essigsäure und Wasser über den Kopf der Kolonne abgetrennt werden.

Die enthaltene Acrylsäure wird als Roh-Acrylsäure über einen Seitenabzug der Kolonne entnommen. Die Schwersieder, wie z.B. oligomere Acrylsäure, fallen im Sumpf der Kolonne an. Die so erhaltene Roh-Acrylsäure enthält im allgemeinen ungefähr 0,1 bis ungefähr 2 Gew.-% Essigsäure und ungefähr 0,5 bis ungefähr 3 % Wasser. Darüber hinaus sind im Sumpf weitere Hochsieder, wie z.B. Reste des inerten hochsiedenden Lösungsmittels und Stabilisatoren enthalten.

Dabei wird im allgemeinen wie folgt vorgegangen:

Das Acrylsäure enthaltende gasförmige Gemisch aus Stufe A wird in den unteren Teil einer Destillationskolonne geleitet, in der die gasförmigen Bestandteile und die Leichtsieder, insbesondere Aldehyde, Essigsäure und Wasser, über den Kopf der Kolonne abgetrennt werden.

Die Acrylsäure wird als Roh-Acrylsäure im unteren Drittel der Destillationskolonne über einen Seitenabzug abgezogen.

Die Hochsieder, hauptsächlich das inerte hochsiedende Lösungsmittel sowie oligomere Acrylsäure, fallen im Sumpf der Destillationskolonne an.

Die für das erfindungsgemäße Verfahren einsetzbaren Destillationskolonnen (Kolonnen) unterliegen keiner besonderen Beschränkung. Grundsätzlich eignen sich alle Kolonnen mit trennwirksamen Einbauten.

Als Kolonneneinbauten kommen alle gängigen Einbauten in Betracht, insbesondere Böden, Packungen und/oder Füllkörper. Von den Böden sind Glockenböden, Siebböden, Ventilböden und/oder Dual-Flow-Böden bevorzugt.

Die Kolonne umfaßt wenigstens eine Kühlvorrichtung. Hierfür eignen sich alle Wärmeüberträger oder Wärmetauscher, bei denen die bei der Kondensation frei werdende Wärme indirekt (extern) abgeführt wird. Hierfür können alle gängigen Apparate eingesetzt werden, wobei Rohrbündelwärmetauscher, Plattenwärmetauscher und Luftkühler bevorzugt sind. Geeignete Kühlmedien sind bei einem Luftkühler entsprechend Luft und bei anderen Kühlvorrichtungen Kühlflüssigkeiten, insbesondere Wasser. Ist nur eine Kühlvorrichtung vorgesehen, so wird diese am Kopf der Kolonne eingebaut, in dem die Leichtsiederfraktion auskondensiert wird.

Der Fachmann kann in Abhängigkeit von der gewünschten Reinheit der kondensierten Fraktionen und damit der Komponenten die Anzahl der erforderlichen Kühlvorrichtungen leicht bestimmen, wobei die Reinheit der kondensierten Komponenten im wesentlichen durch die installierte Trennleistung der Kolonne, d.h. die Kolonnenhöhe, die Anzahl der Böden und die über das zu kondensierende gasförmige Gemisch aus Stufe A eingetragene Energie bestimmt wird. Zweckmäßigerweise werden bei Vorhandensein mehrerer Kühlvorrichtungen diese in verschiedenen Abschnitten der Kolonne eingebaut.

Z.B. können bei einem gasförmigen Gemisch aus Stufe A, das neben dem hohen Anteil nicht kondensierbarer Komponenten eine Schwersieder-, Mittelsieder- und Leichtsiederfraktion enthält, eine Kühlvorrichtung im unteren Abschnitt der Kolonne zur Auskondensation der Schwersiederfraktion und eine Kühlvorrichtung am Kopf der Kolonne zur Auskondensation der Leichtsiederfraktion vorgesehen sein. Die kondensierten Fraktionen werden an den jeweiligen Abschnitten in der Kolonne über Seitenabzüge abgeführt. In Abhängigkeit von der Anzahl der Komponenten in der Schwersieder-, Mittelsieder- und Leichtsiederfraktion können jeweils auch mehrere Seitenabzüge vorgesehen sein. Die über die Seitenabzüge abgezogenen Fraktionen können dann weiteren Reinigungsstufen unterzogen werden, z.B. destillativen oder extraktiven Trennverfahren oder einer Kristallisation, je nach gewünschter Reinheit der Komponenten.

In einer bevorzugten Ausgestaltung der Erfindung sind ein Schwersiederabzug, ein Leichtsiederabzug und 1 oder 2 Mittelsiederabzüge vorgesehen.

Der in der Kolonne vorliegende Druck hängt von der Menge an nicht kondensierbaren Komponenten ab und beträgt vorzugsweise 0,5 - 5 bar Absolutdruck, insbesondere 0,8 - 3 bar Absolutdruck.

Die Temperatur im Bereich der Auftrennvorrichtung, in dem die Leichtsieder, d.h. im wesentlichen typischerweise Aldehyde, Essigsäure und Wasser, abgetrennt werden, beträgt ungefähr 25 bis ungefähr 50 °C, vorzugsweise ungefähr 30 bis ungefähr 40 °C; die Temperatur im Bereich, in dem die Roh-Acrylsäure erhalten wird, beträgt ungefähr 70 bis 110 °C, vorzugsweise ungefähr 80 bis 100 °C. Die Sumpftemperatur wird bei ungefähr 90 bis ungefähr 140 °C, insbesondere bei ungefähr 115 bis 135 °C gehalten.

Die genauen Betriebsbedingungen für die Kolonne, wie Temperatur- und Druckführung, Schaltung und Anordnung der Kühlvorrichtung(en), Anordnung der Seitenabzüge zum Abziehen der gewünschten Fraktionen, Wahl der Kolonnenhöhe und des Kolonnendurchmessers, Anzahl und Abstand der trennwirksamen Einbauten/Böden in der Kolonne sowie die Art der trennwirksamen Kolonneneinbauten, können vom Fachmann im Rahmen fachüblicher Versuche in Abhängigkeit von der Trennaufgabe ermittelt werden.

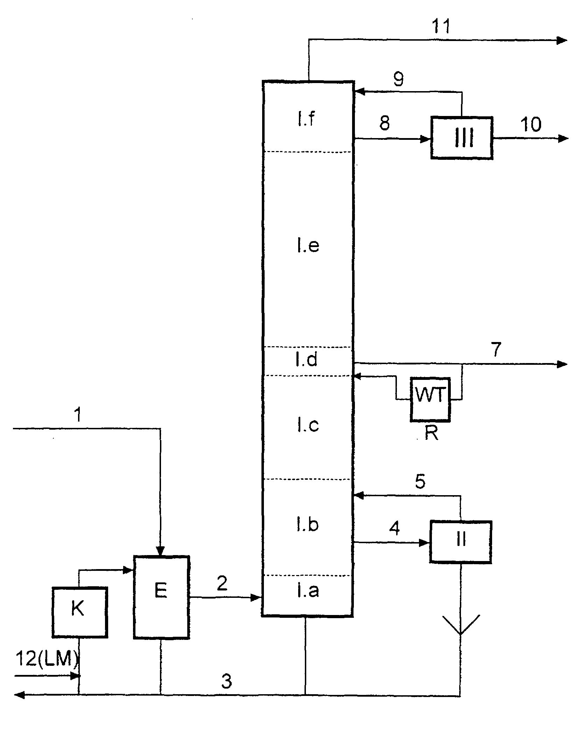

Vorteilhafterweise wird das Verfahren bei Vorhandensein einer Schwersiederfraktion, einer Mittelsiederfraktion, einer Leichtsiederfraktion und nicht kondensierbarer Komponente(n) in dem Acrylsäure enthaltenden, gasförmigen Gemisch (gasförmiges Gemisch) so durchgeführt, wie es in der Figur gezeigt ist und wie es im folgenden beschrieben ist, wobei sich die Kolonne in verschiedene Abschnitte untergliedern läßt, in denen unterschiedliche verfahrenstechnische Aufgaben gelöst werden.

Die Bezugsziffern in der Figur bezeichnen hierbei die einzelnen Abschnitte in der Kolonne (I.a bis I.f) bzw. separate Abschnitte/Apparate vor der Kolonne (E), Zu- und Ableitungen (1 - 12) sowie die Kühlkreise II und III.

### E. Quench:

### Abkühlung des gasförmigen Gemischs

In der Einrichtung E wird das gasförmige Gemisch eingeleitet und abgekühlt. Dies kann über indirekte Kühlung, mit einem inerten hochsiedenden Lösungsmittel (LM) als Kühlmedium, das über Zuleitung (12) zugeführt werden kann, erfolgen. Dabei wird das gasförmige Gemisch aus Leitung 1 in einem Quench E abgekühlt und über Leitung 2 dem Sumpfbereich I.a der Kolonne zugeführt. Über Leitung 3 wird die kondensierte Schwersiederfraktion zusammen mit dem inerten hochsiedenden Lösungsmittel vermischt und dem Kühlkreislauf zugeführt und in den Quench zurückgeführt. Dabei kann das zur Kühlung zurückgeführte Gemisch aus Schwersiederfraktion und inertem hochsiedendem Lösungsmittel in einer Kühlvorrichtung (K) abgekühlt, vorzugsweise auf 80 - 150 °C abgekühlt werden. Ein Teil des Stromes, üblicherweise 0,1 bis 10 Gew.-% bezogen auf 100 Gew.-% der gewonnenen Acrylsäure, wird aus dem Prozeß ausgeschleust und teilweise durch frisches Lösungsmittel über Zuleitung 12 ersetzt.

### I.b Kühlkreis II:

### Kondensation der Schwersiederfraktion

Im Kolonnenabschnitt I.b wird die Kondensationswärme extern über Kühlkreis II mittels eines Wärmetauschers mit z.B. Wasser als Kühlmedium abgeführt, indem kondensierte Schwersiederfraktion über Leitung 4 aus der Kolonne abgeführt wird, gekühlt wird und ein Teil der gekühlten, kondensierten Schwersiederfraktion über Leitung 5 der Kolonne rückgeführt wird, während ein anderer Teil, entsprechend dem Stand im Quench, über Leitung 3 ausgeschleust bzw. in den Quench E zurückgeführt wird.

### I.c Kühlkreis II → Seitenabzug:

### Schwersiederanreichung

Im Kolonnenabschnitt I.c zwischen Kolonnenabschnitt I.b (Kühlkreis II) und I.d (Seitenabzug) erfolgt zum Kühlkreis II hin eine destillative Anreicherung und Auskondensation der Schwersiederfraktion aus dem im Gegenstrom nach oben geführten gasförmigen Gemisch.

### I.d Seitenabzug:

### Abziehen der Mittelsiederfraktion

Über Seitenabzug 7 im Kolonnenabschnitt I.d. wird die gewünschte Zielkomponente Acrylsäure als Roh-Acrylsäure z.B. über einen Fangboden flüssig abgeführt und teilweise als Rücklauf (R) unterhalb des Seitenabzugs 7 über einen Wärmetauscher (WT) geleitet und in die Kolonne zurückgeführt.

### I.e Seitenabzug → Kühlkreis III:

### Mittelsiederanreicherung

Im Kolonnenabschnitt I.e zwischen Kolonnenabschnitt I.d (Seitenabzug 7) und I.f (Kühlkreis III) erfolgt die destillative Anreicherung der Mittelsiederfraktion des gasförmigen Gemischs aus dem nach oben geführten gasförmigen Gemisch, wobei die Mittelsiederfraktion zum Seitenabzug (Bereich I.d) hin angereichert wird.

### I.f Kühlkreis III:

### Kondensation der Leichtsiederfraktion

Im Kolonnenabschnitt I.f des externen Kühlkreises III erfolgt die Kondensation der Leichtsiederfraktion aus dem im Gegenstrom nach oben geführten gasförmigen Gemisch. Analog zu Kühlkreis II wird die Kondensationswärme extern über Kühlkreis III mittels eines Wärmetauschers (nicht gezeigt) mit z.B. Wasser als Kühlmedium abgeführt, indem kondensierte Leichtsiederfraktion über Leitung 8 abgezogen und gekühlt wird und ein Teil der gekühlten, kondensierten Leichtsiederfraktion über Leitung 9 der Kolonne rückgeführt wird, während der andere Teil über Leitung 10 ausgeschleust wird. Die nicht kondensierten Gase werden vom Kopf der Kolonne über Leitung 11 abgezogen, wobei ggf. der Gasstrom noch überhitzt werden kann, um eine weitere Kondensation im Brüdenrohr zu vermeiden.

Das Gas wird vorzugsweise als Kreisgas über Leitung 11 in die Acrylsäure-Herstellung zurückgefahren.

Weitere Details bezüglich dieser Verfahrensweise sind der DE-A 197 40 253 zu entnehmen, deren Inhalt durch Bezugnahme vollumfänglich in den Kontext der vorliegenden Anmeldung aufgenommen wird.

Während des Auftrennens wird zur Stabilisierung ein Polymerisationsinhibitor, wie z.B. Phenothiazin, eine phenolische Verbindung, eine N-O-Verbindung oder ein Gemisch aus zwei oder mehr davon, vorzugsweise Phenothiazin oder Hydrochinon, ein Gemisch aus Phenothiazin und Hydrochinon, Hydrochinonmonomethylether, p-Nitrosophenol, Nitrosodiethylanilin oder Tetramethylpiperidin-1-oxylen, wie sie in der DE-A-16 18 141 beschrieben sind, zugegeben.

Die nach der Auftrennung erhaltenen Leichtsieder, also im wesentlichen Wasser und Essigsäure werden nach dem Ausschleusen aus der Auftrennvorrichtung ganz oder teilweise, gegebenenfalls unter Zugabe eines Polymerisationsinhibitors als Rücklauf wieder in den oberen Teil der Auftrennvorrichtung zurückgeführt, um dort die Kondensation der im Acrylsäure enthaltenen gasförmigen Gemisch enthaltenen Leichtsieder zu erleichtern.

Die als Mittelsieder vorzugsweise über einen Seitenabzug erhaltene Roh-Acrylsäure wird ganz oder teilweise der Kristallisation bzw. Destillation nach einem aus dem Stand der Technik bekannten Verfahren unterworfen, wobei eine Rein-Acrylsäure erhalten wird. Dabei wird Mutterlauge aus der Kristallisation ganz oder teilweise und/oder ein Teil der Roh-Acrylsäure unterhalb des Seitenabzugs der Kolonne zugeführt.

Darüber hinaus kann die erfindungsgemäß erhaltene Roh-Acrylsäure auch einer Veresterung mit einem Alkanol unterworfen werden.

Demgemäß betrifft die vorliegende Erfindung auch ein Verfahren zur Herstellung eines Acrylsäureesters oder eines Gemischs aus zwei oder mehr davon, das zusätzlich zu den oben definierten Stufen A und B eine weitere Stufe C umfaßt:
C: Veresterung der in Stufe B erhaltenen Roh-Acrylsäure mittels einem oder mehrerer Alkanole, wobei ein Veresterungsgemisch umfassend einen oder mehrere Acrylsäureester und einen oder mehrere Essigsäureester erhalten wird.

Weitere Details bezüglich der Veresterung von Acrylsäure lassen sich der DE-A 195 47 485 und den darin zitierten Stand der Technik entnehmen, die voll umfänglich in den Kontext der vorliegenden Anmeldung durch Bezugnahme einbezogen wird.

Femer betrifft sie ein Verfahren, das zusätzlich zu der Stufe A bis C eine weitere Stufe D umfaßt:
D: Auftrennung des Veresterungsgemisches unter Erhalt eines oder mehrerer Acrylsäureester, sowie eines Auftrennungsgemisches, das einen oder mehrere Essigsäureester umfaßt.

In einer bevorzugten Ausführungsform wird das erfindungsgemäße Verfahren zur Acrylsäure-Herstellung wie folgt durchgeführt.

Die Roh-Acrylsäure wird direkt mit einem C₁-C₁₂, vorzugsweise C₁-C₁₀-Alkanol, insbesondere C₄-C₈-Alkanol verestert. Dabei wird die Veresterung nach einem Verfahren des Standes der Technik, z.B. dem Verfahren der DE-A 195 47 485, durchgeführt, wobei die Veresterungsbedingungen vom verwendeten Alkanol abhängig sind.

Als bevorzugte Alkanole sind zu nennen:
Methanol, Ethanol, iso-Propanol, n-Butanol, iso-Butanol, Octanol, 2-Ethylhexanol, vorzugsweise n-Butanol, iso-Butanol und 2-Ethylhexanol.

Typische Bedingungen, unter denen die Veresterung stattfinden kann, sind wie folgt:
Verhältnis Alkanol:Acrylsäure:
   1:0,7-1,2 (molar)
Katalysator:
   Schwefelsäure oder eine Sulfonsäure, wie z.B. p-Toluolsulfonsäure
Katalysatormenge:
   Ungefähr 0,1 - 10 Gew.-%, vorzugsweise ungefähr 0,5 - 5 Gew.-%, jeweils bezogen auf die Einsatzstoffe
Stabilisator:
   Phenothiazin, Hydrochinon, Hydrochinonmonomethylether, Phenylendiamin und ggf. Luft
Stabilisatormenge:
   Ungefähr 100 bis ungefähr 50.000 ppm, vorzugsweise ungefähr 500 bis ungefähr 2.000 ppm, jeweils bezogen auf Acrylsäure.
Umsetzungstemperatur:
   Ungefähr 80 - 160 °C, vorzugsweise ungefähr 90 - 130 °C
Druck während der Umsetzung:
   0,5 - 1,5 bar, vorzugsweise bei Atmosphärendruck
Umsetzungsdauer:
   Ungefähr 1 bis ungefähr 10 Stunden, vorzugsweise ungefähr 1 bis 6 Stunden.

Ggf. kann ein Schleppmittel, wie z.B. Cyclohexan oder Toluol zur Entfernung des während der Veresterung entstehenden Wassers eingesetzt werden.

Die Veresterung an sich kann drucklos, mit Überdruck oder Unterdruck, sowie kontinuierlich als auch diskontinuierlich durchgeführt werden, wobei eine kontinuierliche Führung des Gesamtverfahrens, d.h. eine kontinuierliche Durchführung der hierin erläuterten Stufen A bis D bevorzugt ist.

Da erfindungsgemäß die in Stufe B erhaltene Roh-Acrylsäure verestert wird, erhält man ein Veresterungsgemisch, das den (die) gewünschten Acrylsäureester und darüber hinaus den (die) entsprechenden Essigsäureester umfaßt.

Die Isolierung der Acrylsäureester erfolgt auf herkömmliche Weise. In der Regel werden dabei zunächst der Katalysator und die nicht umgesetzte Acrylsäure ausgewaschen und anschließend das Veresterungsgemisch aufgetrennt, vorzugsweise destillativ aufgetrennt.

Bei dieser Auftrennung wird einerseits der eine oder mehrere Acrylsäureester und andererseits ein Auftrennungsgemisch, das einen oder mehrere Essigsäureester umfaßt, erhalten. Dieses Auftrennungsgemisch wird vorzugsweise in einer weiteren Stufe E verseift, wobei ein Verseifungsgemisch erhalten wird, das ein oder mehrere Alkanole und Essigsäuresalze enthält. Aus diesem Verseifungsgemisch kann anschließend das Alkanol wiederum abgetrennt werden.

### Im einzelnen wird dabei vorzugsweise wie folgt vorgegangen:

Zunächst wird das Veresterungsgemisch destillativ aufgetrennt, wobei eine Leichtsiederfraktion, die u.a. den Essigsäureester umfaßt und ein Sumpfprodukt, das die Hauptmenge des Acrylsäureesters enthält, erhalten wird. Das Sumpfprodukt wird danach ebenfalls destillativ aufgetrennt, wobei der Acrylsäureester über Kopf erhalten wird.

Die bei der destillativen Auftrennung anfallende Leichtsiederfraktion, die hauptsächlich aus Alkanol (ungefähr 20 bis ungefähr 70 %), Essigsäureester (ungefähr 5 bis ungefähr 40 %) und Acrylsäureester (ungefähr 5 bis ungefähr 50 %) besteht, wird ggf. zusammen mit dem bei der Reindestillation des gewünschten Esters anfallenden Destillationssumpf, der u.a. Diacrylsäureester, Alkoxypropionsäureester und oligomere und polymere Acrylsäureester enthält, mit einer 5 bis 40 gew.-%igen wäßrigen Alkalilauge, vorzugsweise NaOH bei Siedetemperatur ungefähr 30 min bis ungefähr 10 Stunden lang behandelt.

Die Leichtsiederfraktion kann dabei ggf. noch in einem weiteren Auftrennungs-, vorzugsweise Destillationsschritt in ein hauptsächlich aus Alkanol und Acetat bestehendes Kopfprodukt und ein Sumpfprodukt, das im wesentlichen aus Acrylsäureester besteht, aufgetrennt werden. Das dabei erhaltene Kopfprodukt wird dann wie vorstehend ausgeführt verseift. Der erhaltene Acrylsäureester wird vorzugsweise der destillativen Aufarbeitung des Veresterungsgemisches zugeführt.

Die Umsetzung mit Alkalilauge (Verseifung) kann kontinuierlich oder diskontinuierlich, drucklos oder bei Über- oder Unterdruck durchgeführt werden. Vorzugsweise wird dazu ein Rühr- oder Rohrreaktor eingesetzt.

Die Abtrennung des Alkanols aus dem erhaltenen Verseifungsgemisch hängt von der Art des Alkanols, d.h. von dessen Wasserlöslichkeit, ab. In Wasser unlösliche Alkanole bilden eine zweite Phase aus und können einfach abgetrennt werden. In Wasser lösliche Alkanole werden z.B. durch Destillation bzw. durch Strippen mit Luft oder Wasserdampf abgetrennt. Vorzugsweise werden die erhaltenen Alkanole dann wiederum der Veresterung zugeführt. Die destillative Abtrennung oder das Strippen kann beispielsweise in einem beheizbaren Rührreaktor mit einer aufgesetzten Kolonne erfolgen. Der Energieeintrag kann auf herkömmliche Weise erfolgen (Doppelwand-, Rohrschlangenheizung, Umlauferhitzer usw.).

Auch das Strippen des Alkanols in einer Strippkolonne kann auf übliche Weise erfolgen. Beispielsweise kann die heiße (ungefähr 40 bis ungefähr 80 °C) Verseifungslösung am Kopf einer Kolonne eingespeist werden und im Gegenstrom mit Luft (ungefähr 1 bis ungefähr 20 m³/m³) oder Dampf (ungefähr 0,1 bis ungefähr 10 t/m³) gestrippt werden. Die Kondensation des Alkanols aus dem Strippgas kann mit einem herkömmlichen Kühler, z.b. einem Rohrbündel- oder Plattenwärmeaustauscher, erfolgen.

Das Alkanol kann dann wieder der Veresterung gemäß Stufe C zugeführt werden.

Das erfindungsgemäße Verfahren weist die folgenden Vorteile auf:
1. Wasserarme Roh-Acrylsäure kann technisch einfach gewonnen werden. Es wird nur eine Auftrennvorrichtung, vorzugsweise eine Destillationskolonne benötigt.
2. Bedingt durch eine geringe Verschmutzung durch Polymerisat weist die verwendete Anlage eine lange Laufzeit auf.
3. Wegen der Rückgewinnung der Alkanolkomponente aus den bei der Veresterung anfallenden Essigsäureestern werden Verluste an Alkanol auf ein Minimum beschränkt.

In ihrer allgemeinsten Ausführungsform betrifft die vorliegende Erfindung ferner die Verwendung eines inerten hochsiedenden Lösungsmittels zum Abkühlen eines bei der Gasphasenoxidation zur Herstellung von Acrylsäure anfallenden gasförmigen Reaktionsgemischs, das Acrylsäure enthält.

Die vorliegende Erfindung soll nunmehr anhand eines Beispiels erläutert werden.

### Beispiel

Durch zweistufige katalytische Oxidation von Propylen mit molekularem Sauerstoff wurde auf übliche Weise ein gasförmiges Reaktionsgemisch mit folgender Zusammensetzung erhalten:
9,84 Gew. - % Acrylsäure,
0,4 Gew.-% Essigsäure,
4,42 Gew.-% Wasser,
0,11 Gew.-% Acrolein,
0,21 Gew.-% Formaldehyd,
0,07 Gew.-% Maleinsäureanhydrid, sowie
Propionsäure, Furfural, Propan, Propen, Stickstoff, Sauerstoff und Kohlenoxide.

Dieses gasförmige Reaktionsgemisch wurde in einem Sprühkühler (Quench) durch Eindüsen eines eutektischen Gemischs aus Diphenyl und Diphenylether (0,5 m³/m³) auf 140°C abgekühlt. Die Roh-Acrylsäure wurde dabei über einen Wärmeaustauscher im Kreis gefahren und eine Temperatur von 95 °C eingestellt.

Das abgekühlte, gasförmige Gemisch, das Acrylsäure enthielt, wurde über einen Tropfenabscheider (Zyklon) in den unteren Teil einer Destillationskolonne geleitet, die mit 60 Dual-Flow-Böden, einem Seitenabzug zwischen dem 15. und 16. Boden und einem Sprühkondensator am Kopf der Kolonne ausgerüstet war. Die Temperatur am Kopf der Destillationskolonne betrug 34 °C, die Sumpftemperatur der Destillationskolonne betrug 118 °C.

Das im Sprühkondensator anfallende Destillat, das hauptsächlich aus Wasser und Essigsäure bestand, wurde nach Ausschleusung von 20 % desselben und Zugabe von 500 ppm Hydrochinon als Rücklauf wieder auf den obersten Kolonnenboden aufgebracht.

Der Sumpf der Destillationskolonne wurde über einen Wärmeaustauscher, wobei eine Temperatur von 95 °C eingestellt wurde, wieder dem 5. Kolonnenboden zugeführt.

Die über den Seitenabzug ausgeschleuste Roh-Acrylsäure enthielt 96,6 Gew.-% Acrylsäure, 1,2 Gew.-% Essigsäure, 0,05 Gew.-% Propionsäure und 1,5 Gew.-% Wasser.

Auf den 20. Boden wurden pro Stunde 100 ml mit 2000 ppm Phenothiazin stabilisierte Roh-Acrylsäure zudosiert.

Eine Rührkesselkaskade bestehend aus drei Rührreaktoren mit je einem Liter Reaktionsvolumen, die mit Kolonne, Kondensator und Phasentrenngefäß ausgerüstet waren, wurden mit 500 g der aus dem Quench ausgetragenen Roh-Acrylsäure, 570 g Butanol und 13 g Schwefelsäure pro Stunde beschickt. Die Reaktionstemperatur in den Reaktoren betrug 106 °C, 118 °C und 123 °C; der Druck betrug jeweils 700 mbar. Am Kopf der Kolonne fiel ein Gemisch aus Wasser, Butanol und Butylacrylat an, das in eine wäßrige und eine organische Phase zerfiel. Die organische Phase wurde mit 300 ppm Phenothiazin versetzt und der Kolonne als Rücklauf zugeführt.

Der Reaktoraustrag (945 g/h) wurde auf 30 °C abgekühlt, die nicht umgesetzte Acrylsäure und der Katalysator mit 5 %iger Natronlauge neutralisiert, mit Wasser gewaschen und anschließend in einer Siebbodenkolonne mit 60 Böden destilliert. Der Zulauf zur Kolonne erfolgte auf dem 5. Boden. Die Sumpftemperatur betrug 110 °C, die Kopftemperatur betrug 88 °C; der Druck betrug 160 mbar.

Am Kopf dieser Kolonne fielen 850 g/h Destillat an, das sich in eine organische Phase (841 g/h) und eine Wasserphase trennte. 748 g/h der organischen Phase wurden mit 300 ppm Phenothiazin versetzt und als Rücklauf wieder auf den obersten Boden der Siebbodenkolonne aufgebracht.

Die organische Phase des Destillats enthielt 12,3 % Essigsäurebutylester, 41,7 % Butanol und 43,6 % Butylacrylat.

Der Sumpfablauf wurde in einer weiteren Siebbodenkolonne (30 Böden) in Butylacrylat mit einer Reinheit von 99,7 % (735 g/h) als Kopfprodukt, sowie Hochsieder, die hauptsächlich Inhibitoren und oligomeres/polymeres Butylacrylat enthielten, als Sumpfprodukt aufgetrennt. Bei dieser Trennung betrug die Sumpftemperatur 108°C, die Kopftemperatur 80°C, bei einem Druck von 100 mbar. Das Rücklaufverhältnis betrug 0,6.

In einem Rührreaktor wurde ein Gemisch aus 1.000 g der organischen Phase des Destillats mit 40 %-iger Natronlauge (800 g) zwei Stunden lang unter Rückfluß erhitzt. Nach Beendigung der Verseifüngsreaktion wurde das gebildete Butanol unter Vakuum (500 mbar) über eine Kolonne (10 Glockenböden) aus dem Reaktor destillativ abgetrennt. Das Kondensat zerfiel in eine Wasserphase und eine Butanolphase (812 g). Die Butanolphase wurde direkt wieder der Veresterung zugeführt. Dabei konnten ungefähr 94 % der theoretischen Butanolmenge zurückgewonnen werden.

## Patentansprüche

1. Verfahren zur Herstellung von Acrylsäure, das folgende Stufe A umfaßt:
A: Abkühlen eines bei der Gasphasenoxidation zur Herstellung von Acrylsäure anfallenden gasförmigen Reaktionsgemisches, das Acrylsäure enthält, mittels eines inerten hochsiedenden Lösungsmittels, ***das hierbei nicht verdampft*,** wobei ein Acrylsäure enthaltendes, gasförmiges Gemisch erhalten wird.

2. Verfahren nach Anspruch 1, **dadurch gekennzeichnet, daß** in Stufe A das gasförmige Reaktionsgemisch, das Acrylsäure enthält, auf eine Temperatur von 120 bis 180 °C abgekühlt wird.

3. Verfahren nach Anspruch 1 oder 2, **dadurch gekennzeichnet, daß** in Stufe A das gasförmige Reaktionsgemisch, das Acrylsäure enthält, in einem Sprühkühler abgekühlt wird.

4. Verfahren nach einem der Ansprüche 1 bis 3, das folgende weitere Stufe B umfaßt:
B: Auftrennung des Acrylsäure enthaltenden, gasförmigen Gemischs, wobei eine Leichtsiederfraktion, eine Roh-Acrylsäure und ein Sumpfprodukt erhalten wird.

5. Verfahren nach Anspruch 4, **dadurch gekennzeichnet, daß** die Auftrennung gemäß Stufe B in einer Destillationskolonne durchgeführt wird und die Roh-Acrylsäure über einen Seitenabzug der Destillationskolonne erhalten wird.

6. Verfahren nach einem der Ansprüche 1 bis 5, **dadurch gekennzeichnet, daß** die in Stufe B erhaltene Roh-Acrylsäure in Rein-Acrylsäure überführt wird.

7. Verfahren zur Herstellung eines Acrylsäureesters oder eines Gemischs aus zwei oder mehr davon, **dadurch gekennzeichnet, daß** es eine Stufe A, wie in einem der Ansprüche 1 bis 3 definiert, eine Stufe B, wie in Anspruch 4 oder 5 definiert, sowie eine weitere Stufe C umfaßt:
C: Veresterung der in Stufe B erhaltenen Roh-Acrylsäure mittels einem oder mehreren Alkanolen, wobei ein Veresterungsgemisch umfassend einen oder mehrere Acrylsäureester und einen oder mehrere Essigsäureester erhalten wird.

8. Verfahren nach Anspruch 7, **dadurch gekennzeichnet, daß** es eine weitere Stufe D umfaßt:
D: Auftrennung des Veresterungsgemisches unter Erhalt eines oder mehrerer Acrylsäureester, sowie eines Auftrennungsgemisches, das einen oder mehrere Essigsäureester umfaßt.

9. Verfahren nach Anspruch 8, **dadurch gekennzeichnet, daß** es eine weitere Stufe E umfaßt:
E: Verseifung des Auftrennungsgemisches, wobei ein Verseifungsgemisch, das ein oder mehrere Alkanole und Essigsäuresalze enthält, erhalten wird.

10. Verfahren nach Anspruch 9, **dadurch gekennzeichnet, daß** ein oder mehrere Alkanole aus dem Verseifungsgemisch abgetrennt werden und in die Veresterung gemäß Stufe C zurückgeführt werden.

11. Verwendung eines inerten hochsiedenden Lösungsmittels zum Abkühlen eines bei der Gasphasenoxidation zur Herstellung von Acrylsäure anfallenden gasförmigen Reaktionsgemisches, das Acrylsäure enthält.

## Claims

1. A process for preparing acrylic acid comprising the following stage A:
A: cooling a gaseous reaction mixture which comprises acrylic acid and is obtained in the gas-phase oxidation to prepare acrylic acid, using an inert high-boiling solvent which does not evaporate in this stage, to give a gaseous mixture comprising acrylic acid.

2. A process as claimed in claim 1, wherein the gaseous reaction mixture comprising acrylic acid is cooled in stage A to a temperature of from 120 to 180°C.

3. A process as claimed in claim 1 or 2, wherein the gaseous reaction mixture comprising acrylic acid is cooled in stage A in a spray cooler.

4. A process as claimed in any one of claims 1 to 3, which comprises the following further stage B:
B: separating the gaseous mixture comprising acrylic acid to give a low-boiling fraction, a crude acrylic acid, and a bottom product.

5. A process as claimed in claim 4, wherein the separation of stage B is conducted in a distillation column and the crude acrylic acid is obtained via a sidestream takeoff of the distillation column.

6. A process as claimed in any one of claims 1 to 5, wherein the crude acrylic acid obtained in stage B is converted to pure acrylic acid.

7. A process for preparing an acrylate, or a mixture of two or more thereof, which comprises a stage A as defined in any one of claims 1 to 3, a stage B as defined in claim 4 or 5, and a further stage C:
C: esterifying the crude acrylic acid obtained in stage B by means of one or more alkanols to give an esterification mixture comprising one or more acrylates and one or more acetic esters.

8. A process as claimed in claim 7, which comprises a further stage D:
D: separating the esterification mixture to give one or more acrylates and a separation mixture which comprises one or more acetic esters.

9. A process as claimed in claim 8, which comprises a further stage E:
E: hydrolyzing the separation mixture to give a hydrolysis mixture which comprises one or more alkanols and acetic salts.

10. A process as claimed in claim 9, wherein one or more alkanols are separated from the hydrolysis mixture and recycled to the esterification of stage C.

11. The use of an inert high-boiling solvent for cooling a gaseous reaction mixture comprising acrylic acid and obtained in the gas-phase oxidation to prepare acrylic acid.

## Revendications

1. Procédé de préparation d'acide acrylique, comprenant l'étape A suivante:
A: Refroidissement d'un mélange réactionnel gazeux provenant de l'oxydation en phase gazeuse pour la préparation d'acide acrylique, contenant de l'acide acrylique, au moyen d'un solvant inerte à haut point d'ébullition, sans évaporation de ce solvant, avec obtention d'un mélange gazeux contenant de l'acide acrylique.

2. Procédé selon la revendication 1, **caractérisé en ce que** dans l'étape A, le mélange réactionnel gazeux, contenant de l'acide acrylique, est refroidi à une température de 120 à 180°C.

3. Procédé selon la revendication 1 ou 2, **caractérisé en ce que** dans l'étape A, le mélange réactionnel, contenant de l'acide acrylique, est refroidi dans un refroidisseur à pulvérisation.

4. Procédé selon l'une des revendications 1 à 3, comprenant l'étape subséquente B suivante:
B: Séparation du mélange gazeux contenant de l'acide acrylique, avec obtention d'une fraction de tête volatile, d'un acide acrylique brut et d'un produit de fond de colonne.

5. Procédé selon la revendication 4, **caractérisé en ce que** la séparation selon l'étape B est entreprise dans une colonne de distillation et que l'acide acrylique brut est obtenu par un soutirage latéral de la colonne de distillation.

6. Procédé selon l'une des revendications 1 à 5, **caractérisé en ce que** l'acide acrylique brut obtenu à l'étape B est converti en acide acrylique pur.

7. Procédé de préparation d'un ester d'acide acrylique ou d'un mélange de deux ou plus de deux d'entre eux, **caractérisé en ce qu'**il comprend une étape A, comme définie dans l'une des revendications 1 à 3, une étape B, comme définie à la revendication 4 ou 5, et une autre étape C:
C: Estérification de l'acide acrylique brut obtenu à l'étape B, au moyen d'un ou plusieurs alcanol(s), avec obtention d'un mélange d'estérification contenant un ou plusieurs ester(s) d'acide acrylique et un ou plusieurs ester(s) d'acide acétique.

8. Procédé selon la revendication 7, **caractérisé en ce qu'**il contient une autre étape subséquente D:
D: Séparation du mélange d'estérification avec obtention d'un ou plusieurs ester(s) d'acide acrylique, ainsi qu'un mélange de séparation contenant un ou plusieurs ester(s) d'acide acétique.

9. Procédé selon la revendication 8, **caractérisé en ce qu'**il comprend une autre étape subséquente E:
E: Saponification du mélange de séparation, avec obtention d'un mélange de saponification contenant un ou plusieurs alcanol(s) et de l'acide acétique.

10. Procédé selon la revendication 9, **caractérisé en ce que** l'on sépare du mélange de saponification un ou plusieurs alcanol(s) et qu'on les recycle dans l'estérification selon l'étape C.

11. Utilisation d'un solvant inerte à haut point d'ébullition pour le refroidissement d'un mélange réactionnel gazeux contenant de l'acide acrylique, provenant de l'oxydation en phase gazeuse pour la préparation d'acide acrylique.
